# EUROPÄISCHE PATENTANMELDUNG

(11) **EP 4 736 905 A1**
(43) Veröffentlichungstag der Anmeldung: **06.05.2026**
(21) Anmeldenummer: 25210916.0
(22) Anmeldetag: 23.10.2025
(51) Int. Cl.: A61M 1/16

(54) **VERFAHREN ZUR DICHTIGKEITSPRÜFUNG EINES WÄRMETAUSCHERS EINER BLUTBEHANDLUNGSVORRICHTUNG**

(30) Priorität: 29.10.2024 DE 102024131576
(71) Anmelder: B. Braun Avitum AG, 34212 Melsungen (DE)
(72) Erfinder: JANIK, Waldemar, 34212 Melsungen (DE); KRAUSE, Silvie, 34212 Melsungen (DE)
(74) Vertreter: Maiwald GmbH

(57) **Zusammenfassung**

Die Erfindung betrifft ein Verfahren zur Dichtigkeitsprüfung eines Wärmetauschers einer Blutbehandlungsvorrichtung, umfassend Überwachen des zeitlichen Verlaufs eines mittels eines Drucksensors erfassten Druckes in einem ersten Abschnitt eines Flüssigkeitskreislaufs der Blutbehandlungsvorrichtung in einem Bewertungszeitraum; Überprüfen mittels eines Rechensystems, ob der zeitliche Verlauf des Druckes in dem Bewertungszeitraum ein oder mehrere vorbestimmte Kriterien erfüllt; und Feststellen einer Undichtigkeit des Wärmetauschers für den Fall, dass der zeitliche Verlauf das bzw. die vorbestimmten Kriterien erfüllt, wobei das Überwachen des zeitlichen Verlaufs in dem Bewertungszeitraum das Überwachen des zeitlichen Verlaufs während eines Fördervorrichtungsbetriebs zum Druckaufbau/Unterdruckaufbau und/oder nach einem Druckaufbau/Unterdruckaufbau umfasst.

## Beschreibung

### TECHNISCHES GEBIET

Die Erfindung betrifft ein Verfahren zur Dichtigkeitsprüfung eines Wärmetauschers einer Blutbehandlungsvorrichtung und eine Blutbehandlungsvorrichtung und System mit dieser.

### HINTERGRUND DER ERFINDUNG

Blutbehandlungsvorrichtungen, beispielsweise Dialysesysteme, nutzen in der Regel einen Wärmetauscher, der die Wärme der abfließenden Flüssigkeit, beispielsweise des Dialysats, nutzt, um das zufließende und im Vergleich zur abfließenden Flüssigkeit kältere Permeat vorzuwärmen. Undichtigkeit im Wärmetauscher werden in derzeitigen Blutbehandlungsvorrichtungen nicht erkannt. Vielmehr wird davon ausgegangen, dass es zu keinen unbemerkten Undichtigkeiten kommt.

Somit besteht die Gefahr, dass eine Kontamination, beispielsweise durch Übergang von abfließender Flüssigkeit, beispielsweise verbrauchtem Dialysat, in zufließende Flüssigkeit, beispielsweise frisches Permeat, bzw. auf die Dialysierflüssigkeitsseite, nicht erkannt wird.

Der Erfindung liegt die Aufgabe zugrunde, ein Verfahren bereitzustellen, das zumindest manche der oben ausgeführten Probleme adressiert, insbesondere eine zuverlässige Erkennung von Undichtigkeit des Wärmetauschers ermöglicht.

### ZUSAMMENFASSUNG DER ERFINDUNG

Die Erfindung stellt ein, insbesondere computer-implementiertes, Verfahren zur Dichtigkeitsprüfung eines Wärmetauschers einer Blutbehandlungsvorrichtung, eine Blutbehandlungsvorrichtung und ein System gemäß den unabhängigen Ansprüchen bereit. Spezielle Ausführungsformen finden sich in den abhängigen Ansprüchen.

Die Erfindung betrifft ein, insbesondere computer-implementiertes, Verfahren zur Dichtigkeitsprüfung eines Wärmetauschers einer Blutbehandlungsvorrichtung, umfassend: Überwachen des zeitlichen Verlaufs eines mittels eines Drucksensors erfassten Druckes in einem ersten Abschnitt eines Flüssigkeitskreislaufs der Blutbehandlungsvorrichtung in einem Bewertungszeitraum; Überprüfen mittels eines Rechensystems, ob der zeitliche Verlauf des Druckes in dem Bewertungszeitraum ein oder mehrere vorbestimmte Kriterien erfüllt; und Feststellen einer Undichtigkeit des Wärmetauschers für den Fall, dass der zeitliche Verlauf das bzw. die vorbestimmten Kriterien erfüllt.

Der Wärmetauscher weist zwei Strömungswege umfassend einen ersten Strömungsweg, der mit dem ersten Abschnitt des Flüssigkeitskreislaufs fluidisch verbunden ist, und einen zweiten Strömungsweg, der mit einem zweiten Abschnitt des Flüssigkeitskreislaufs fluidisch verbunden ist, auf. Der Flüssigkeitskreislauf ist über die Strömungswege mit einem Zulauf und einen Ablauf verbunden.

Der erste Abschnitt des Flüssigkeitskreislaufs ist während des Bewertungszeitraums unter Verwendung von einem oder mehreren Schaltelementen von dem zweiten Abschnitt des Flüssigkeitskreislaufs, dem Zulauf und/oder dem Ablauf fluidisch getrennt.

Der Bereich des Flüssigkeitskreislaufs, der mit dem Zulauf verbunden ist, wird als zulaufseitiger Bereich des Flüssigkeitskreislaufs bezeichnet. Der Bereich des Flüssigkeitskreislaufs, der mit dem Ablauf verbunden ist, wird als ablaufseitiger Bereich des Flüssigkeitskreislaufs bezeichnet. Der erste Abschnitt, also der Abschnitt, in dem der Druck überwacht wird, kann, im zulaufseitigen oder im ablaufseitigen Bereich sein.

In der vorliegenden Offenbarung wird häufig davon gesprochen, dass der erste Abschnitt bzw. der zweite Abschnitt vom jeweils anderen Abschnitt, dem Zulauf und/oder dem Ablauf fluidisch getrennt sind/werden. Soweit hier nicht anders spezifiziert, sind der erste Abschnitt und der zweite Abschnitt auch dann jeweils mit dem ersten Strömungsweg bzw. zweiten Strömungsweg des Wärmetauschers fluidisch verbunden.

In der vorliegenden Offenbarung wird davon ausgegangen, dass bei fluidisch miteinander verbundenen Bereichen/Abschnitten, sofern es nicht explizit anders angegeben wird, die fluidische Verbindung offen bzw. nicht getrennt ist.

Das Überwachen des zeitlichen Verlaufs in dem Bewertungszeitraum umfasst das Überwachen des zeitlichen Verlaufs in einem ersten Bewertungszeitraum während eines Fördervorrichtungsbetriebs zum Druckaufbau/Unterdruckaufbau in dem ersten Abschnitt und/oder dem zweiten Abschnitt des Flüssigkeitskreislaufs und/oder das Überwachen des zeitlichen Verlaufs in einem zweiten Bewertungszeitraum nach einem Druckaufbau/Unterdruckaufbau in dem ersten Abschnitt des Flüssigkeitskreislaufs.

Es kann mit anderen Worten also ein Verfahren bereitgestellt werden, bei dem systematisch die Dichtigkeit eines Wärmetauschers geprüft wird, insbesondere geprüft wird, ob eine Undichtigkeit vorliegt, bei der abfließende Flüssigkeit in die zufließende Flüssigkeit übertritt. Dazu wird ein Drucksensor verwendet, der in einem bestimmten Abschnitt den Druck überwacht.

Wenn an bestimmten Stellen des Flüssigkeitskreislaufs durch Fördervorrichtungsbetrieb ein Druck oder Unterdruck aufgebaut wird, dann wird dieser sich nicht wie erwartet verhalten, wenn auf Grund einer Undichtigkeit im Wärmetauscher keine vollständige fluidische Trennung zwischen dem ersten und dem zweiten Abschnitt im Bereich des Wärmetauschers vorliegt. Ähnliches gilt auch nach dem Aufbau eines Drucks oder Unterdrucks, beispielsweise wenn der Druckwert ungewöhnlich stark und/oder schnell absinkt bzw. ansteigt.

Die Undichtigkeit kann sich an verschiedenen Abschnitten im Flüssigkeitskreislauf nachweisen lassen, die unter den Anspruch 1 fallen und besonders auch in den bevorzugten Ausführungsformen beispielhaft näher spezifiziert werden.

Beispielsweise kann Flüssigkeit aus dem Zulauf durch den Wärmetauscher und einen oder beide Abschnitte gefördert werden, jedenfalls aber in den ersten Abschnitt. Dazu kann, wenn der erste Abschnitt zulaufseitig ist, der erste vom stromabwärtsgelegenen zweiten Abschnitt getrennt werden. Wenn der erste Abschnitt ablaufseitig ist, kann dazu der erste Abschnitt vom Ablauf getrennt werden (nach dem Wärmetauscher). Dadurch baut sich ein Druck (zumindest) im ersten Abschnitt auf. Wenn ein Teil der zugeführten Flüssigkeit durch einen undichten Wärmetauscher aber wieder zulaufseitig in den Flüssigkeitskreislauf gelangt, wird sicher der Druck langsamer aufbauen.

In einem weiteren Beispiel kann, nachdem ein Druck im ersten Abschnitt aufgebaut wurde, der erste Abschnitt, beispielsweise mittels Absperrelementen, vom zweiten Abschnitt getrennt werden und vom Ablauf bzw. Zulauf getrennt werden bzw. bleiben. Damit ist der erste Abschnitt (zusammen mit dem ersten Strömungsweg des Wärmetauschers) im wesentlichen fluidisch isoliert, wenn der Wärmetauscher dicht ist. Der Druck im ersten Abschnitt wird im Falle eines undichten Wärmetauschers schneller und/oder stärker absinken als im Fall eines dichten Wärmetauschers, weil Flüssigkeit aus dem ersten Abschnitt in den zweiten Abschnitt entweicht.

In einem weiteren Beispiel können der erste Abschnitt und der zweite Abschnitt voneinander getrennt sein und im zweiten Abschnitt kann ein Druck aufgebaut werden. Dies sollte im Wesentlichen keine Auswirkung auf den davon abgetrennten ersten Abschnitt haben. Wenn aber der Wärmetauscher undicht ist, so wird sich dort der Druck dennoch verändern.

Der Flüssigkeitskreislauf kann derart in mindestens zwei Abschnitte unterteilt werden, dass die Abschnitte nur dann fluidisch vollständig voneinander trennbar sind, wenn der Wärmetauscher dicht ist.

Zur Terminologie sei hier angemerkt, dass in der vorliegenden Offenbarung immer derjenige Abschnitt, in dem der Verlauf des erfassten Druckes überwacht wird, als "erster Abschnitt" bezeichnet wird. Das heißt, dass der erste Abschnitt abhängig davon ist, wo im Flüssigkeitskreislauf die Überwachung erfolgt. Somit kann es auch mehrere erste Abschnitte geben, wenn eine Überwachung in mehreren Abschnitten erfolgt. So können beispielsweise auch mehrere Kriterien verwendet werden, was unten im Detail beschrieben wird.

Der erste Abschnitt kann also jeweils der Abschnitt sein, der ganz oder teilweise fluidisch abgetrennt wird und in dem der Druck gemessen wird. Das können je nach Bewertungszeitraum und Ausführungsform unterschiedliche Bereiche des Flüssigkeitskreislaufs sein, insbesondere ein zulaufseitiger oder ein ablaufseitiger Bereich.

Es kann sich bei der Dichtigkeit insbesondere um die Dichtigkeit zwischen einem ersten Strömungsweg im Wärmetauscher, beispielsweise einem Strömungsweg der zufließenden Flüssigkeit, und einem zweiten Strömungsweg im Wärmetauscher, beispielsweise einem Strömungsweg der abfließenden Flüssigkeit, handeln. Wenn die Dichtigkeit nicht gegeben ist, also eine Undichtigkeit vorliegt, kann Flüssigkeit zwischen dem ersten und dem zweiten Strömungsweg ausgetauscht werden. Insbesondere kann abfließende Flüssigkeit in den Strömungsweg der zufließenden Flüssigkeit übertreten. Dieses Szenario ist bei Blutbehandlungsvorrichtungen äußerst kritisch, da schädliche Stoffe zu einem Patienten gelangen können. Beispielsweise bei einer Dialyse wird zudem die Effizienz gemindert.

Die beanspruche Dichtigkeitsprüfung ermöglicht ein zuverlässiges Erkennen von Undichtigkeit, insbesondere der soeben beschriebenen Undichtigkeit.

Im Normalbetrieb wird die Flüssigkeit derart transportiert, dass sie durch den Wärmetauscher einem Flüssigkeitskreislauf der Blutbehandlungsvorrichtung zufließt und durch den Wärmetauscher aus dem Flüssigkeitskreislauf abfließt.

Wie oben beschrieben, weist der Wärmetauscher zwei Strömungswege umfassend einen ersten Strömungsweg und einen zweiten Strömungsweg auf. Diese sind, wenn der Wärmetauscher dicht ist, fluidisch voneinander getrennt. Ein Wärmetausch kann zwischen Flüssigkeit in dem ersten Strömungsweg und Flüssigkeit in dem zweiten Strömungsweg erfolgen. Wenn der Wärmetauscher dicht ist, erfolgt dies, ohne dass die Flüssigkeiten der beiden Strömungswege miteinander in Verbindung kommen. Bei einer Undichtigkeit kommt es zu Flüssigkeitsaustausch zwischen den beiden Strömungswegen.

Die Strömungswege im Wärmetauscher können derart ausgebildet sein, dass ein Wärmetausch zwischen zufließender und abfließender Flüssigkeit erfolgen kann. Im Normalbetrieb kann insbesondere die zufließende Flüssigkeit kälter als die abfließende Flüssigkeit sein, so dass im Wärmetauscher Wärme von der abfließenden Flüssigkeit auf die zufließende Flüssigkeit übertragen wird. Während der Dichtigkeitsprüfung muss jedoch nicht zwingend eine Temperaturdifferenz vorliegen bzw. Wärmetausch stattfinden.

Der Wärmetauscher kann beispielsweise ein Röhrenwärmetauscher, ein Plattenwärmetauscher, ein Spiralwärmetauscher, ein Rohrbündelwärmetauscher, ein Mantelrohrwärmetauscher, oder Hybridformen daraus sein, wobei die Offenbarung nicht auf diese Beispiele beschränkt ist.

Der erste Strömungsweg ist mit dem ersten Abschnitt des Flüssigkeitskreislaufs fluidisch verbunden. Der zweite Strömungsweg ist der mit einem zweiten Abschnitt des Flüssigkeitskreislaufs fluidisch verbunden. Der Flüssigkeitskreislauf ist über die Strömungswege mit einem Zulauf und einen Ablauf verbunden ist.

Das heißt, wenn der erste und der zweite Abschnitt des Flüssigkeitskreislaufs fluidisch getrennt sind, beispielsweise durch ein Absperrelement, wie ein Ventil, kann nur bei Undichtigkeit des Wärmetauschers ein Flüssigkeitsaustausch zwischen dem ersten und dem zweiten Abschnitt auftreten.

Hier sei angemerkt, dass "fluidisch verbunden" in der vorliegenden Offenbarung auch umfassen soll, dass grundsätzlich eine absperrbare Verbindung vorliegt, beispielsweise durch ein Absperrelement, z.B. ein Ventil, absperrbar/trennbar. Wenn eine solche Verbindung abgesperrt ist, beispielsweise durch ein Absperrelement, dann wird in der vorliegenden Offenbarung von "fluidisch getrennt" gesprochen.

Das Transportieren der Flüssigkeit kann beispielsweise mittels einer oder mehrerer Pumpen erfolgen, wobei andere Transportverfahren ebenfalls denkbar sind.

Das Überwachen des zeitlichen Verlaufs des mittels des Drucksensors erfassten Druckes kann umfassen, mittels des Drucksensors Messwerte zu mehreren Zeitpunkten zu erfassen und optional zu verarbeiten. Das Verarbeiten kann beispielsweise Ableiten der Eigenschaft aus den Messwerten, Vorverarbeitung von Rohmesswerten, und/oder Bestimmen einer Funktion, die den Zeitverlauf des Druckes repräsentiert, umfassen.

Gemäß der vorliegenden Offenbarung kann das Überwachen des zeitlichen Verlaufs in einem ersten Bewertungszeitraumwährend eines Fördervorrichtungsbetriebs zum Druckaufbau/Unterdruckaufbau in dem ersten Abschnitt und/oder zweiten Abschnitt erfolgen. Alternativ oder zusätzlich kann das Überwachen des zeitlichen Verlaufs in einem zweiten Bewertungszeitraum nach einem Druckaufbau/Unterdruckaufbau in dem ersten Abschnitt des Flüssigkeitskreislaufs umfassen. Der Bewertungszeitraum kann den ersten und/oder den zweiten Bewertungszeitraum umfassen.

Der Bewertungszeitraum kann grundsätzlich den jeweiligen Umständen entsprechend gewählt werden. Beispielsweise kann der Bewertungszeitraum, insbesondere der erste Bewertungszeitraum, nur einen Teil einer Druckaufbauphase bzw. Unterdruckaufbauphase oder die gesamte Druckaufbauphase bzw. Unterdruckaufbauphase umfassen. Alternativ oder zusätzlich kann der Bewertungszeitraum, insbesondere der zweite Bewertungszeitraum unmittelbar nach dem Druckaufbau/Unterdruckaufbau oder mit einer bestimmten Verzögerung beginnen. Der Bewertungszeitraum kann auch ein Ende einer Druckaufbauphase bzw. Unterdruckaufbauphase und den daran anschließenden Zeitraum umfassen.

Wie oben bereits erwähnt wurde, ist der erste Abschnitt des Flüssigkeitskreislaufs während des Bewertungszeitraums unter Verwendung von einem oder mehreren Schaltelementen von dem zweiten Abschnitt des Flüssigkeitskreislaufs, dem Zulauf und/oder dem Ablauf fluidisch getrennt.

Neben Schaltelementen kann auch das Fluidsystem selbst zu der fluidischen Trennung beitragen. Bei einem dichten Wärmetauscher trägt insbesondere auch die fluidische Trennung zwischen den beiden Strömungswegen zu der fluidischen Trennung des ersten Abschnitts von dem zweiten Abschnitt des Flüssigkeitskreislaufs, dem Zulauf und/oder dem Ablauf bei.

Wie oben gesehen, wird im Bewertungszeitraum der erste Abschnitt zumindest teilweise fluidisch abgetrennt (vom zweiten Abschnitt und/oder, je nachdem, ob er Zulauf oder ablaufseitig ist, vom Zulauf bzw. Ablauf). Das führt dazu, dass eine sehr genaue Vorhersage/Erwartung darüber möglich ist, wie der Druck sich in dem ersten Abschnitt im Bewertungszeitraum verhalten sollte, wenn der Wärmetauscher dicht ist. Bei Undichtigkeit des Wärmetauschers wird der erste Abschnitt nicht mehr in demselben Maß abgetrennt sein, wie bei einem dichten Wärmetauscher. Somit kann sich aus einer Abweichung von der Erwartung einfach und zugleich genau eine Undichtigkeit ableiten lassen.

Wie unten im Detail anhand der Beispiele erläutert wird, kann diese fluidische Trennung gegenüber unterschiedlichen Bereichen erfolgen, beispielsweise abhängig davon, wo und/oder wann der Drucksensor den Druck überwacht.

Bei dem Drucksensor kann es sich um jeden geeigneten bekannten Drucksensor handeln, der den Druck, beispielsweise direkt oder indirekt erfasst.

Wie oben gesehen umfasst das Verfahren ein Überprüfen, ob der zeitliche Verlauf des Druckes in dem Bewertungszeitraum ein oder mehrere vorbestimmte Kriterien erfüllt. Die Kriterien können umfassen, dass ein Druckanstieg und/oder Druckabfall stärker oder schwächer (z.B. steiler und/oder um einen größeren Betrag oder flacher und/oder um einen kleineren Betrag) ist im Vergleich zu einem Verlauf ohne Undichtigkeit. Zu den Kriterien werden unten noch detaillierte Beispiele vorgestellt.

Wie auch aus der Beschreibung der Beispiele unten hervorgeht, können diese Kriterien eine Undichtigkeit, insbesondere ein Übertreten von abfließender Flüssigkeit in die zufließende Flüssigkeit, anzeigen.

Gemäß der vorliegenden Offenbarung umfasst das Verfahren ein Feststellen einer Undichtigkeit des Wärmetauschers für den Fall, dass der zeitliche Verlauf das bzw. die vorbestimmten Kriterien erfüllt.

Das Feststellen der Undichtigkeit kann optional eine Ausgabe einer Warnung und/oder ein automatisches Einleiten von Sicherheitsmaßnahmen auslösen.

Wenn das Feststellen der Undichtigkeit (nur) für den Fall erfolgt, dass mehrere Kriterien erfüllt sind, so können diese Kriterien sich auf den zeitlichen Verlauf des Drucks in einem einzigen ersten Abschnitt beziehen, beispielsweise einen zeitlichen Verlauf beim Druckaufbau/Unterdruckaufbau und einen zeitlichen Verlauf nach dem Druckaufbau/Unterdruckaufbau oder mehrere Kriterien betreffend den Verlauf, beispielsweise Steigung der Druckänderung und absolute Druckänderung. Die Kriterien können sich alternativ oder zusätzlich auf den Druck in verschiedenen ersten Abschnitten beziehen, der beispielsweise mit mehreren Sensoren überwacht werden kann.

Die Verwendung mehrerer Kriterien kann verschiedene Vorteile haben, je nachdem, wie die Kriterien eingesetzt werden. Beispielsweise kann sie eine Redundanz ermöglichen, beispielsweise indem es genügt, dass nur ein Teil, beispielsweise nur eines, der Kriterien erfüllt ist, um eine Undichtigkeit festzustellen. Die Robustheit der Dichtigkeitsprüfung kann erhöht werden, beispielsweise indem nur dann, wenn mehrere Kriterien erfüllt sind, eine Undichtigkeit festgestellt wird, insbesondere alle Kriterien. Die Sicherheit kann erhöht werden, wenn eine Dichtigkeit nur positiv festgestellt wird, wenn keines der Kriterien erfüllt ist. Dies kann eine Zusatzfunktion zusätzlich zum Feststellen einer Undichtigkeit darstellen.

Die Kriterien sind vorgegeben und können beispielsweise anhand von vorhergesagten Verläufen und/oder empirisch festgestellten Verläufen abgeleitet werden, insbesondere auch im Vergleich zu den Verläufen für einen dichten Wärmetauscher. Beispiele dazu finden sich unten. Es kann sich um fallabhängige Kriterien handeln, die der Fachmann im Einzelfall geeignet anpassen wird, insbesondere wenn quantitative Kriterien verwendet werden, die von der Systemauslegung abhängen.

Das Rechensystem kann ein oder mehrere Recheneinrichtungen umfassen, insbesondere eine verteiltes Rechensystem sein. Das Rechensystem kann lokale Computer und/oder Server und/oder mobile Benutzerendgeräte umfassen.

In einem Aspekt der vorliegenden Offenbarung kann der erste Abschnitt des Flüssigkeitskreislaufs in dem Bewertungszeitraum unter Verwendung von einem oder mehreren Schaltelementen von dem zweiten Abschnitt des Flüssigkeitskreislaufs und dem Ablauf fluidisch getrennt sein. Der zweite Abschnitt kann dabei insbesondere fluidisch mit dem Zulauf verbunden sein.

Insbesondere kann der erste Abschnitt zwischen dem zweiten Abschnitt und dem Ablauf angeordnet sein und von beiden fluidisch getrennt sein, wobei der erste Abschnitt weiterhin mit dem ersten Strömungsweg des Wärmetauschers fluidisch verbunden ist, die Absperrung also in Strömungsrichtung hinter dem Wärmetauscher ist. Bei dichtem Wärmetauscher wäre der erste Abschnitt zusammen mit dem ersten Strömungsweg des Wärmetauschers also komplett isoliert.

In diesem Aspekt kann das Überwachen in dem zweiten Bewertungszeitraum durchgeführt werden. Beispielsweise kann das Überwachen nach dem Druckaufbau bzw. Unterdruckaufbau erfolgen. Das vorbestimmte Kriterium kann umfassen, dass ein Druckabfall bzw. Druckanstieg des erfassten Druckes im zweiten Bewertungszeitraum von einem Referenzdruckabfall bzw. Referenzdruckanstieg abweicht, wobei der Druckabfall bzw. Druckanstieg insbesondere größer ist als der Referenzdruckabfall bzw. Referenzdruckanstieg. Insbesondere kann der Betrag oder die Steigung des Druckabfalls bzw. Druckanstiegs von dem Referenzdruckabfall bzw. Druckanstieg abweichen.

Bei einem dichten Wärmetauscher würde nach Druckaufbau der Druck auf eine bestimmte Art und Weise abfallen, beispielsweise mit einer bestimmten Steigung und/oder um einen bestimmten Betrag. Wenn eine Undichtigkeit im Wärmetauscher vorliegt, würde der Druckabfall anders aussehen, insbesondere schneller oder um einen größeren Betrag abfallen. Dies gilt analog auch für den Druckanstieg, nachdem ein Unterdruck aufgebaut wurde. Der Druck bzw. Unterdruck wird beispielsweise weniger lange gehalten.

Mit anderen Worten würde trotz Isolierung der Druck sich unerwartet schnell und/oder stark verändern. Dies ist ein Hinweis auf eine Undichtigkeit im Wärmetauscher.

Alternativ oder zusätzlich kann bei diesem Aspekt das Überwachen in dem ersten Bewertungszeitraum auch während eines Fördervorrichtungsbetriebs einer im ersten Abschnitt angeordneten ersten Fördervorrichtung und/oder einer im zweiten Abschnitt angeordneten zweiten Fördervorrichtung zum Druckaufbau/Unterdruckaufbau in dem ersten Abschnitt durchgeführt werden. Das vorbestimmte Kriterium kann umfassen, dass ein Druckanstieg/Druckabfall des erfassten Druckes im ersten Bewertungszeitraum von einem Referenzdruckanstieg/Referenzdruckabfall abweicht, wobei der Druckanstieg/Druckabfall insbesondere kleiner ist als der Referenzdruckanstieg/Referenzdruckabfall.

Ähnlich wie für den Druckanstieg bzw. Druckabfall nach dem Druckaufbau bzw. Unterdruckaufbau beschrieben, ist bei einem dichten Wärmetauscher während des Druckaufbaus bzw. Unterdruckaufbaus ein bestimmter Referenzdruckanstieg bzw. Referenzdruckabfall zu rechnen. Bei einer Undichtigkeit kann aber beispielsweise der Druck bzw. Unterdruck weniger effizient aufgebaut werden, so dass sich eine Abweichung, insbesondere ein langsamerer Aufbau von Druck bzw. Unterdruck ergibt.

So kann eine Undichtigkeit einfach und zuverlässig nachgewiesen werden.

Der oben beschriebene Druckaufbau/Unterdruckaufbau kann bei fluidisch verbundenem ersten und zweiten Abschnitt erfolgen.

Alternativ kann auch bei fluidischer Trennung der beiden Abschnitte voneinander ein Druck/Unterdruck in einem der beiden Abschnitte, aufgebaut werden. In dem jeweils anderen Abschnitt sollte sich der Druck/Unterdruck bei dichtem Wärmetauscher dabei nicht (nennenswert) verändern. Wenn er dies doch tut, ist das ein Hinweis auf eine Undichtigkeit.

Gemäß der vorliegenden Offenbarung kann der erste Abschnitt des Flüssigkeitskreislaufs in dem Bewertungszeitraum unter Verwendung von einem oder mehreren Schaltelementen von dem zweiten Abschnitt des Flüssigkeitskreislaufs und dem Ablauf fluidisch getrennt sein.

So kann, bei einem dichten Wärmetauscher, der erste Abschnitt fluidisch isoliert werden. Ein Druckaufbau oder Unterdruckaufbau im zweiten Abschnitt wird sich bei einem dichten Wärmetauscher in der oben beschriebenen Konfiguration wenig oder nicht auf den ersten Abschnitt auswirken.

In dieser Konfiguration kann das Überwachen in dem ersten Bewertungszeitraum während eines Fördervorrichtungsbetriebs einer im zweiten Abschnitt angeordneten zweiten Fördervorrichtung zum Druckaufbau/Unterdruckaufbau in dem zweiten Abschnitt durchgeführt werden. Das vorbestimmte Kriterium kann umfassen, dass ein Druckanstieg/Druckabfall des erfassten Druckes (wie oben beschrieben handelt es sich um den im ersten Abschnitt erfassten Druck) im ersten Bewertungszeitraum von einem Referenzdruckanstieg/Referenzdruckabfall abweicht, wobei der Druckanstieg/Druckabfall insbesondere größer ist als der Referenzdruckanstieg/Referenzdruckabfall. Mit anderen Worten wirkt sich eine Druckänderung im zweiten Abschnitt anders, insbesondere mehr, auf den Druck im ersten Abschnitt aus als für einen dichten Wärmetauscher erwartet wird.

In den obigen Konfigurationen kann der erste Abschnitt bezogen auf den zweiten Abschnitt stromabwärts angeordnet sein.

Die Fördervorrichtung kann bezogen auf den Drucksensor stromaufwärts oder stromabwärts angeordnet sein. Alternativ oder zusätzlich kann die erste Fördervorrichtung bezogen auf den Drucksensor stromabwärts angeordnet sein. Auch eine stromaufwärts angeordnete Fördervorrichtung ist jedoch möglich.

In einer weiteren Konfiguration kann der erste Abschnitt des Flüssigkeitskreislaufs in dem Bewertungszeitraum unter Verwendung von einem oder mehreren Schaltelementen von dem zweiten Abschnitt des Flüssigkeitskreislaufs und dem Zulauf fluidisch getrennt sein und insbesondere der zweite Abschnitt fluidisch mit dem Ablauf verbunden sein. Das heißt, in diesem Fall kann der erste Abschnitt in Strömungsrichtung vor dem zweiten Abschnitt angeordnet sein, insbesondere zwischen Zulauf und zweitem Abschnitt. Die obigen Überlegungen, Merkmale und Vorteile zu der Konfiguration, in der zweite Abschnitt in Strömungsrichtung vor dem ersten Abschnitt angeordnet ist, lassen sich jedoch analog übertragen. Letztlich ist dadurch nur vertauscht, mit welchem der Strömungswege des Wärmetauschers der jeweilige Abschnitt verbunden ist.

Das Überwachen kann in dieser Konfiguration in dem zweiten Bewertungszeitraum durchgeführt werden und das vorbestimmte Kriterium kann umfassen, dass ein Druckabfall/Druckanstieg des erfassten Druckes im zweiten Bewertungszeitraum von einem Referenzdruckabfall/Referenzdruckanstieg abweicht, wobei der Druckabfall bzw. Druckanstieg insbesondere größer ist als der Referenzdruckabfall. Mit anderen Worten kann eine Undichtigkeit erkannt werden, wenn der Druck sich mehr verändert, als bei einem dichten Wärmetauscher zu erwarten wäre. Dazu sei auf die obigen Ausführungen verwiesen.

Alternativ oder zusätzlich kann das Überwachen in dem ersten Bewertungszeitraum während eines Fördervorrichtungsbetriebs einer im ersten Abschnitt angeordneten zweiten Fördervorrichtung zum Druckaufbau/Unterdruckaufbau in dem ersten Abschnitt durchgeführt wird. Das vorbestimmte Kriterium kann umfassen, dass ein Druckanstieg/Druckabfall des erfassten Druckes im ersten Bewertungszeitraum von einem Referenzdruckanstieg/Referenzdruckabfall abweicht, wobei der Druckanstieg/Druckabfall insbesondere kleiner ist als der Referenzdruckanstieg/Referenzdruckabfall. Mit anderen Worten kann eine Undichtigkeit erkannt werden, wenn ein Druckaufbau bzw. Unterdruckaufbau nicht wie erwartet erfolgt, insbesondere langsamer erfolgt. Auch dazu sei auf die obigen Ausführungen verwiesen.

Wie oben kurz erwähnt kann in dieser Konfiguration der erste Abschnitt bezogen auf den zweiten Abschnitt stromaufwärts angeordnet sein.

Die zweite Fördervorrichtung kann in dieser Konfiguration bezogen auf den Drucksensor beispielsweise stromaufwärts angeordnet sein.

Im oben beschriebenen zweiten Bewertungszeitraum kann der Fördervorrichtungsbetrieb, insbesondere der ersten Fördervorrichtung und/oder der zweiten Fördervorrichtung, eingestellt sein. Das heißt, im zweiten Bewertungszeitraum kann beispielsweise kein weiterer Druckaufbau bzw. Unterdruckaufbau erfolgen. Das reduziert die Fehleranfälligkeit und erhöht die Zuverlässigkeit, weil Referenzdruckaufbau bzw. Referenzdruckabfall weniger Abhängigkeiten haben, beispielsweise nicht von den Fördervorrichtungen und deren Kennlinien abhängen.

Die zweite Fördervorrichtung kann eine oder mehrere Konzentratpumpen umfassen, die zum Zuführen von Konzentrat in eine Flüssigkeit im Flüssigkeitskreislauf ausgebildet sind. Dies ist vorteilhaft, weil so eine bereits bestehende Komponente verwendet werden kann und nicht zusätzliche Bauteile für die Dichtigkeitsprüfung bereitgestellt werden müssen.

Gemäß der vorliegenden Offenbarung können der erste Abschnitt bzw. der zweite Abschnitt mittels eines externen Absperrelements fluidisch vom Ablauf trennbar sein. Das externe Absperrelement kann mittels einer Steuervorrichtung der Blutbehandlungsvorrichtung und/oder mittels einer externen Steuervorrichtung steuerbar sein, wobei die externe Steuervorrichtung zum Steuern des externen Absperrelements und optional der Blutbehandlungsvorrichtung ausgebildet ist. Eine derartige Steuerung ermöglicht eine Koordination der verschiedenen Komponenten derart, dass die Ergebnisse der Überwachung des Drucks zuverlässig, insbesondere auch zeitlich präzise, mit der Betätigung von Absperrelementen und/oder Fördervorrichtungen assoziiert werden können. Dadurch ist der Vergleich mit Referenzverläufen zuverlässiger und eine Leckage kann zuverlässiger erkannt werden.

Gemäß der vorliegenden Offenbarung kann ein Zeitpunkt zum Öffnen und/oder Schließen des externen Absperrelements mittels einer Synchronisationsvorrichtung von der Blutbehandlungsvorrichtung an das externe Absperrelement kommuniziert werden. So muss das Absperrelement nicht eigens angesteuert werden und es kann trotzdem der oben im Zusammenhang mit der Steuervorrichtung beschriebene Vorteil erzielt werden.

Die externe Steuervorrichtung kann als eine externe Synchronisationsvorrichtung ausgebildet sein, die insbesondere Daten von der Blutbehandlungsvorrichtung empfängt und basierend darauf eine Ablaufsteuerung durchführt. Die externe Synchronisationsvorrichtung kann insbesondere derart ausgebildet sein, dass sie die gesamte Ablaufsteuerung durchführt. Die externe Synchronisationsvorrichtung kann insbesondere derart ausgebildet sein, dass sie bestimmt, wann das externe Ventil und/oder andere Ventile zu öffnen bzw. zu schließen sind und/oder wann der Bewertungszeitraum beginnt.

Optional können alle Schritte automatisch durchgeführt werden.

Das Verfahren kann umfassen, ein oder mehrere Absperrelemente und/oder ein oder mehrere Fördervorrichtungen, insbesondere automatisch, beispielsweise mittels der Steuervorrichtung zu steuern, so dass die Abschnitte gemäß dem obigen Verfahren fluidisch getrennt werden und ggf. gemäß dem obigen Verfahren ein Druckaufbau bzw. Unterdruckabbau durchgeführt wird.

Die vorliegende Offenbarung stellt auch eine Blutbehandlungsvorrichtung bereit.

Die Blutbehandlungsvorrichtung umfasst:
einen Flüssigkeitskreislauf und einen Wärmetauscher, wobei der Wärmetauscher zwei Strömungswege umfassend einen ersten Strömungsweg, der mit einem ersten Abschnitt des Flüssigkeitskreislaufs fluidisch verbunden ist, und einen zweiten Strömungsweg, der mit einem zweiten Abschnitt des Flüssigkeitskreislaufs fluidisch verbunden ist, aufweist und der Flüssigkeitskreislauf über die Strömungswege mit einem Zulauf und einen Ablauf verbunden ist;
einen Drucksensor, der zum Erfassen eines Druckes in dem ersten Abschnitt des Flüssigkeitskreislaufs der Blutbehandlungsvorrichtung ausgebildet ist;
ein Rechensystem, das zum Überwachen eines zeitlichen Verlaufs des mittels eines Drucksensors erfassten Druckes in einem Bewertungszeitraum, in dem die Blutbehandlungsvorrichtung eine Prüfkonfiguration aufweist, zum Überprüfen, ob der zeitliche Verlauf des Druckes in dem Bewertungszeitraum ein oder mehrere vorbestimmte Kriterien erfüllt, und zum Feststellen einer Undichtigkeit des Wärmetauschers für den Fall, dass der zeitliche Verlauf das bzw. die vorbestimmten Kriterien erfüllt, ausgebildet ist,
wobei der erste Abschnitt des Flüssigkeitskreislaufs in der Prüfkonfiguration unter Verwendung von einem oder mehreren Schaltelementen von dem zweiten Abschnitt des Flüssigkeitskreislaufs, dem Zulauf und/oder dem Ablauf fluidisch getrennt ist, und
wobei das Überwachen des zeitlichen Verlaufs in dem Bewertungszeitraum das Überwachen des zeitlichen Verlaufs in einem ersten Bewertungszeitraum während eines Fördervorrichtungsbetriebs zum Druckaufbau/Unterdruckaufbau in dem ersten Abschnitt und/oder dem zweiten Abschnitt des Flüssigkeitskreislaufs und/oder das Überwachen des zeitlichen Verlaufs in einem zweiten Bewertungszeitraum nach einem Druckaufbau/Unterdruckaufbau in dem ersten Abschnitt des Flüssigkeitskreislaufs umfasst.

Der Begriff "Prüfkonfiguration" kann als die Konfiguration der Blutbehandlungsvorrichtung, insbesondere die fluidischen Verbindungen und/oder Schaltungen der Blutbehandlungsvorrichtung, in einem Prüfungszeitraum, der insbesondere den Überwachungszeitraum enthalten oder mit dem Überwachungszeitraum übereinstimmen kann, betrachtet werden. Die Prüfkonfiguration der Blutbehandlungsvorrichtung kann zumindest die Konfiguration des Flüssigkeitskreislaufs umfassen.

Der Fördervorrichtungsbetrieb zum Druckaufbau kann beispielsweise den Betrieb einer Pumpe umfassen.

Gemäß der vorliegenden Offenbarung kann in einer ersten Prüfkonfiguration der Blutbehandlungsvorrichtung der erste Abschnitt des Flüssigkeitskreislaufs unter Verwendung von einem oder mehreren Schaltelementen von dem zweiten Abschnitt des Flüssigkeitskreislaufs und dem Ablauf fluidisch getrennt sein und insbesondere der zweite Abschnitt fluidisch mit dem Zulauf verbunden sein.

Die Blutbehandlungsvorrichtung kann ausgebildet sein, das Überwachen in dem zweiten Bewertungszeitraum durchzuführen, und das vorbestimmte Kriterium kann umfassen, dass ein Druckabfall bzw. Druckanstieg des erfassten Druckes im zweiten Bewertungszeitraum von einem Referenzdruckabfall bzw. Referenzdruckanstieg abweicht, wobei der Druckabfall/Druckanstieg insbesondere größer ist als der Referenzdruckabfall.

Alternativ oder zusätzlich kann die Blutbehandlungsvorrichtung ausgebildet sein, das Überwachen in dem ersten Bewertungszeitraum während eines Fördervorrichtungsbetriebs einer im ersten Abschnitt angeordneten ersten Fördervorrichtung und/oder einer im zweiten Abschnitt angeordneten zweiten Fördervorrichtung zum Druckaufbau/Unterdruckaufbau in dem ersten Abschnitt durchzuführen, und das vorbestimmte Kriterium kann umfassen, dass ein Druckanstieg/Druckabfall des erfassten Druckes im ersten Bewertungszeitraum von einem Referenzdruckanstieg/Referenzdruckabfall abweicht, wobei der Druckanstieg/Druckabfall insbesondere kleiner ist als der Referenzdruckanstieg/Referenzdruckabfall.

In der ersten Prüfkonfiguration kann insbesondere der erste Abschnitt bezogen auf den zweiten Abschnitt stromabwärts angeordnet sein und/oder die Fördervorrichtung bezogen auf den Drucksensor stromaufwärts oder stromabwärts angeordnet sein und/oder die erste Fördervorrichtung bezogen auf den Drucksensor stromabwärts angeordnet sein.

Gemäß der vorliegenden Offenbarung kann in einer zweiten Prüfkonfiguration der Blutbehandlungsvorrichtung der erste Abschnitt des Flüssigkeitskreislaufs unter Verwendung von einem oder mehreren Schaltelementen von dem zweiten Abschnitt des Flüssigkeitskreislaufs und dem Ablauf fluidisch getrennt sein.

Die Blutbehandlungsvorrichtung kann ausgebildet sein, das Überwachen in dem ersten Bewertungszeitraum während eines Fördervorrichtungsbetriebs einer im zweiten Abschnitt angeordneten zweiten Fördervorrichtung zum Druckaufbau/Unterdruckaufbau in dem zweiten Abschnitt durchzuführen, und das vorbestimmte Kriterium umfasst, dass ein Druckanstieg/Druckabfall des erfassten Druckes im ersten Bewertungszeitraum von einem Referenzdruckanstieg/Referenzdruckabfall abweicht, wobei der Druckanstieg/Druckabfall insbesondere größer ist als der Referenzdruckanstieg/Referenzdruckabfall.

In der zweiten Prüfkonfiguration kann insbesondere der erste Abschnitt bezogen auf den zweiten Abschnitt stromabwärts angeordnet sein.

Gemäß der vorliegenden Offenbarung kann in einer dritten Prüfkonfiguration der Blutbehandlungsvorrichtung der erste Abschnitt des Flüssigkeitskreislaufs unter Verwendung von einem oder mehreren Schaltelementen von dem zweiten Abschnitt des Flüssigkeitskreislaufs und dem Zulauf fluidisch getrennt sein und insbesondere der zweite Abschnitt fluidisch mit dem Ablauf verbunden sein.

Die Blutbehandlungsvorrichtung kann ausgebildet sein, das Überwachen in dem zweiten Bewertungszeitraum durchzuführen, und das vorbestimmte Kriterium umfasst, dass ein Druckabfall des erfassten Druckes im zweiten Bewertungszeitraum von einem Referenzdruckabfall abweicht, wobei der Druckabfall insbesondere größer ist als der Referenzdruckabfall.

Alternativ oder zusätzlich kann die Blutbehandlungsvorrichtung ausgebildet sein, das Überwachen in dem ersten Bewertungszeitraum während eines Fördervorrichtungsbetriebs einer im ersten Abschnitt angeordneten zweiten Fördervorrichtung zum Druckaufbau/Unterdruckaufbau in dem ersten Abschnitt durchzuführen. Das vorbestimmte Kriterium kann umfassen, dass ein Druckanstieg/Druckabfall des erfassten Druckes im ersten Bewertungszeitraum von einem Referenzdruckanstieg/Referenzdruckabfall abweicht, wobei der Druckanstieg/Druckabfall insbesondere kleiner ist als der Referenzdruckanstieg/Referenzdruckabfall.

In der dritten Prüfkonfiguration kann insbesondere der erste Abschnitt bezogen auf den zweiten Abschnitt stromaufwärts angeordnet sein. Die zweite Fördervorrichtung kann alternativ oder zusätzlich bezogen auf den Drucksensor stromaufwärts angeordnet sein. Die Fördervorrichtung kann alternativ oder zusätzlich bezogen auf den Drucksensor stromaufwärts oder stromabwärts angeordnet sein. Die erste Fördervorrichtung kann alternativ oder zusätzlich bezogen auf den Drucksensor stromabwärts angeordnet sein.

Die Blutbehandlungsvorrichtung kann zumindest zum Sammeln, Entgasen und/oder Erwärmen von Flüssigkeit im Rahmen einer extrakorporalen Blutbehandlung ausgebildet sein. Die Blutbehandlungsvorrichtung kann zudem zur Zudosierung von Konzentraten zur Herstellung von Dialysierflüssigkeit und/oder zur Bilanzierung samt Ultrafiltration ausgebildet sein. Die Blutbehandlungsvorrichtung kann eine Blutpumpe, einen Dialysator, einen Drucksensor und/oder ein Schlauchsystem umfassen. Die Blutbehandlungsvorrichtung kann auch einen oder mehrere Aktoren und/oder ein oder mehrere (weitere) Sensoren zur Durchführung einer Dialysebehandlung umfassen.

Die Blutbehandlungsvorrichtung kann zum Durchführen des Verfahrens gemäß der vorliegenden Offenbarung, insbesondere wie oben beschrieben, ausgebildet sein.

Das Verfahren der vorliegenden Offenbarung kann insbesondere unter Verwendung der Blutbehandlungsvorrichtung der vorliegenden Offenbarung, insbesondere wie oben beschrieben, ausgeführt werden.

Die vorliegende Offenbarung betrifft auch ein System umfassend die Blutbehandlungsvorrichtung gemäß der vorliegenden Offenbarung.

Das System kann ein externes Absperrelement und eine Steuervorrichtung der Blutbehandlungsvorrichtung und/oder eine externe Steuervorrichtung umfassen. Der erste Abschnitt bzw. der zweite Abschnitt können mittels des externen Absperrelements fluidisch vom Ablauf trennbar sein, insbesondere wobei das externe Absperrelement mittels der Steuervorrichtung der Blutbehandlungsvorrichtung und/oder mittels der externen Steuervorrichtung steuerbar sein, wobei die externe Steuervorrichtung zum Steuern des externen Absperrelements und optional der Blutbehandlungsvorrichtung ausgebildet ist.

Alternativ oder zusätzlich kann das System ein/das externe Absperrelement und eine Synchronisationsvorrichtung umfassen, wobei ein Zeitpunkt zum Öffnen und/oder Schließen des externen Absperrelements mittels der Synchronisationsvorrichtung von der Blutbehandlungsvorrichtung an das externe Absperrelement kommuniziert wird.

Die Merkmale und Vorteile, die im Zusammenhang mit dem Verfahren beschrieben wurden, gelten entsprechend auch für die Blutbehandlungsvorrichtung und das System.

### KURZE BESCHREIBUNG DER FIGUREN

Weitere Beispiele und Ausführungsformen werden im Folgenden anhand der Figuren erläutert. Dabei zeigen:
- Figur 1: eine schematische Darstellung eines Verfahrens gemäß der vorliegenden Offenbarung;
- Figur 2: eine schematische Darstellung eines Systems gemäß der vorliegenden Offenbarung;
- Figur 3: eine schematische Darstellung eines Systems gemäß der vorliegenden Offenbarung;
- Figur 4: eine schematische Darstellung eines Systems gemäß der vorliegenden Offenbarung;
- Figur 5: eine schematische Darstellung eines zeitlichen Signalverlaufs.

### DETAILLIERTE BESCHREIBUNG DER ERFINDUNG

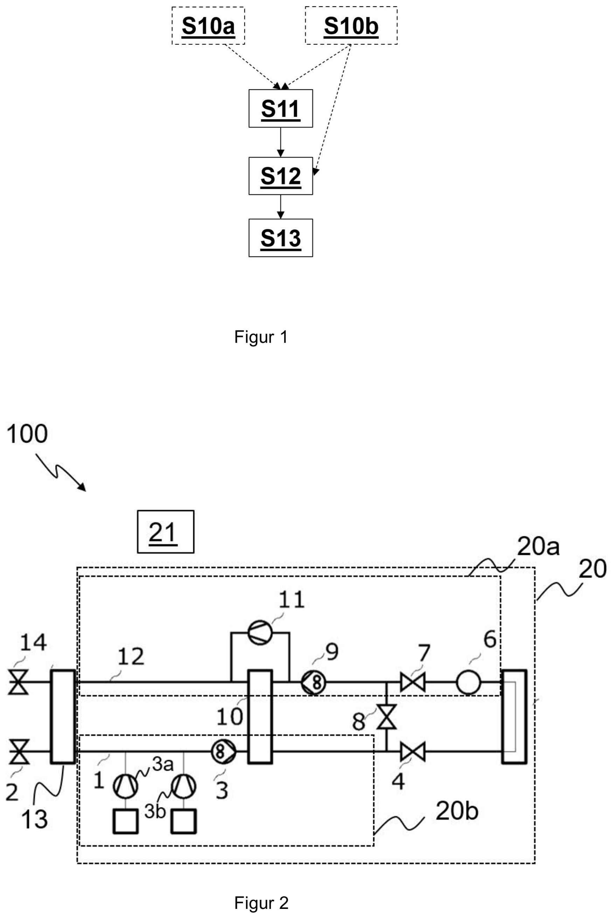

In Figur 1 ist schematisch ein Verfahren zur Dichtigkeitsprüfung eines Wärmetauschers einer Blutbehandlungsvorrichtung gemäß der vorliegenden Offenbarung dargestellt.

Das Verfahren umfasst, in Schritt S11, Überwachen des zeitlichen Verlaufs eines mittels eines Drucksensors erfassten Druckes in einem ersten Abschnitt eines Flüssigkeitskreislaufs der Blutbehandlungsvorrichtung in einem Bewertungszeitraum.

Das Verfahren umfasst, in Schritt S12, Überprüfen mittels eines Rechensystems, ob der zeitliche Verlauf des Druckes in dem Bewertungszeitraum ein oder mehrere vorbestimmte Kriterien erfüllt.

Das Verfahren umfasst, in Schritt S13, Feststellen einer Undichtigkeit des Wärmetauschers für den Fall, dass der zeitliche Verlauf das bzw. die vorbestimmten Kriterien erfüllt.

Der Wärmetauscher weist dabei zwei Strömungswege umfassend einen ersten Strömungsweg, der mit dem ersten Abschnitt des Flüssigkeitskreislaufs fluidisch verbunden ist, und einen zweiten Strömungsweg, der mit einem zweiten Abschnitt des Flüssigkeitskreislaufs fluidisch verbunden ist, auf und der Flüssigkeitskreislauf ist über die Strömungswege mit einem Zulauf und einen Ablauf verbunden.

Der erste Abschnitt des Flüssigkeitskreislaufs ist während des Bewertungszeitraums unter Verwendung von einem oder mehreren Schaltelementen von dem zweiten Abschnitt des Flüssigkeitskreislaufs, dem Zulauf und/oder dem Ablauf fluidisch getrennt.

Das Überwachen des zeitlichen Verlaufs in dem Bewertungszeitraum umfasst das Überwachen des zeitlichen Verlaufs in einem ersten Bewertungszeitraum während eines Fördervorrichtungsbetriebs zum Druckaufbau/Unterdruckaufbau in dem ersten Abschnitt und/oder dem zweiten Abschnitt des Flüssigkeitskreislaufs und/oder das Überwachen des zeitlichen Verlaufs in einem zweiten Bewertungszeitraum nach einem Druckaufbau/Unterdruckaufbau in dem ersten Abschnitt des Flüssigkeitskreislaufs.

In optionalem Schritt S10a können Schaltelemente, beispielsweise Ventile geschaltet werden, um die entsprechenden Schaltungen im Flüssigkeitskreislauf herzustellen.

Dies kann automatisch erfolgen. In optionalem Schritt S10b können Förderelemente, beispielsweise Pumpen, betrieben werden, beispielsweise zum Druckaufbau bzw. Unterdruckaufbau. Dieser Schritt kann beispielsweise vor der Überwachung (beispielsweise im zweiten Bewertungszeitraum) und/oder während der Überwachung (beispielsweise im ersten Bewertungszeitraum) erfolgen.

Das Verfahren der vorliegenden Offenbarung, insbesondere beschrieben im Zusammenhang mit Figur 1, kann beispielsweise mit der Blutbehandlungsvorrichtung gemäß der vorliegenden Offenbarung, insbesondere auch wie in Figur 2 gezeigt, durchgeführt werden. Auch eine Durchführung mit anderen Blutbehandlungsvorrichtungen ist jedoch denkbar.

In Figur 2 ist beispielhaft eine Blutbehandlungsvorrichtung 100 gemäß der vorliegenden Offenbarung dargestellt.

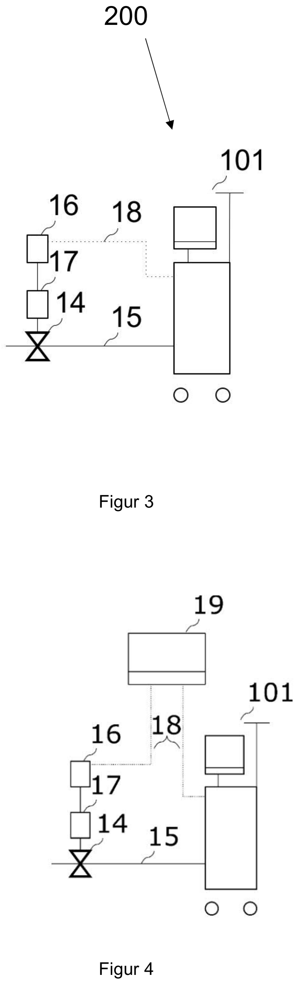

Die Blutbehandlungsvorrichtung umfasst zumindest einen Wärmetauscher 13, einen Flüssigkeitskreislauf 20, einen Sensor 6 und ein Rechensystem 21. Die übrigen in Figur 2 dargestellten Elemente sind optional. Das Rechensystem 21 kann der Kontrollvorrichtung 19 in Figuren 3 oder 4 ganz oder teilweise entsprechen.

Der Wärmetauscher weist zwei Strömungswege umfassend einen ersten Strömungsweg, der mit einem ersten Abschnitt des Flüssigkeitskreislaufs fluidisch verbunden ist, und einen zweiten Strömungsweg, der mit einem zweiten Abschnitt des Flüssigkeitskreislaufs fluidisch verbunden ist, auf. Der Flüssigkeitskreislauf ist über die Strömungswege mit einem Zulauf und einen Ablauf verbunden. In dem Beispiel in Figur 2 ist der Zulauf rein beispielhaft noch durch ein Ventil 2 (auch "Ventil Wassereingang" oder "Eingangsventil") vom Flüssigkeitskreislauf getrennt und der Ablauf durch ein Ventil 14 (auch "Ventil Abfluss" oder "Ausgangsventil") vom Flüssigkeitskreislauf getrennt.

Der Drucksensor 6 ist zum Erfassen eines Druckes in dem ersten Abschnitt des Flüssigkeitskreislaufs 20 der Blutbehandlungsvorrichtung 100 ausgebildet.

Das Rechensystem 21 ist zum Überwachen eines zeitlichen Verlaufs des mittels des Drucksensors 6 erfassten Druckes in einem Bewertungszeitraum, in dem die Blutbehandlungsvorrichtung eine Prüfkonfiguration aufweist, zum Überprüfen, ob der zeitliche Verlauf des Druckes in dem Bewertungszeitraum ein oder mehrere vorbestimmte Kriterien erfüllt, und zum Feststellen einer Undichtigkeit des Wärmetauschers 13 für den Fall, dass der zeitliche Verlauf das bzw. die vorbestimmten Kriterien erfüllt, ausgebildet ist.

Der erste Abschnitt des Flüssigkeitskreislaufs ist in der Prüfkonfiguration unter Verwendung von einem oder mehreren Schaltelementen von dem zweiten Abschnitt des Flüssigkeitskreislaufs, dem Zulauf und/oder dem Ablauf fluidisch getrennt ist.

Das Überwachen des zeitlichen Verlaufs in dem Bewertungszeitraum umfasst das Überwachen des zeitlichen Verlaufs in einem ersten Bewertungszeitraum während eines Fördervorrichtungsbetriebs zum Druckaufbau/Unterdruckaufbau in dem ersten Abschnitt und/oder dem zweiten Abschnitt des Flüssigkeitskreislaufs und/oder das Überwachen des zeitlichen Verlaufs in einem zweiten Bewertungszeitraum nach einem Druckaufbau/Unterdruckaufbau in dem ersten Abschnitt des Flüssigkeitskreislaufs.

Der Flüssigkeitskreislauf kann beispielsweise eine eingangsseitige Leitung 1 umfassen, der über den Wärmetauscher Flüssigkeit, beispielsweise Wasser, zugeführt werden kann. Ein Förderelement 3, beispielsweise eine Flusspumpe, kann im eingangsseitigen Bereich des Flüssigkeitskreislaufs zur Förderung von Flüssigkeit und/oder zum Druck/Unterdruckaufbau vorgesehen sein. Auch Konzentratpumpen 3a und 3b können optional vorgesehen sein. Mit diesen Pumpen kann Konzentrat aus entsprechenden Konzentratquellen in den Flüssigkeitskreislauf gefördert werden, insbesondere im Bereich der eingangsseitigen Leitung. Diese können ebenfalls bei entsprechendem Betrieb verwendet werden, um Flüssigkeit durch den Flüssigkeitskreislauf zu fördern und/oder Druck aufzubauen.

Der Flüssigkeitskreislauf kann beispielsweise eine ausgangsseitige Leitung 12 umfassen, aus der über den Wärmetauscher Flüssigkeit, beispielsweise Dialysat, aus dem Flüssigkeitskreislauf abgeführt werden kann.

Der Wärmetauscher kann so ausgebildet sein, dass Wärme zwischen der durch den Wärmetauscher dem Flüssigkeitskreislauf zugeführten und durch den Wärmetauscher aus dem Flüssigkeitskreislauf abgeführten Flüssigkeit ausgetauscht wird. Bei dichtem Wärmetauscher liegt kein Übertritt von der austretenden in die eintretende Flüssigkeit oder umgekehrt vor.

Ein Förderelement 9, beispielsweise eine Flusspumpe, kann im ausgangsseitigen Bereich des Flüssigkeitskreislaufs zur Förderung von Flüssigkeit und/oder zum Druck/Unterdruckaufbau vorgesehen sein.

Zwischen dem eingangsseitigen Teil und dem ausgangsseitigen Teil des Flüssigkeitskreislaufs können, wie in Figur 2 gezeigt, eine Dialysierbrücke/Spülbrücke 5 mit einem Eingangsventil 4 (auch "Ventil Dialysatoreingang") und einem Ausgangsventil 7 (auch "Ventil Dialysatorausgang") vorgesehen sein. Außerdem kann ein Ventil 8 vorgesehen sein, das als Bypass verwendet werden kann, also um Flüssigkeit an der Spülbrücke vorbeizuleiten.

Weiter können optional eine Bilanzierungsvorrichtung 10 und eine entsprechende Fördervorrichtung 11 vorgesehen sein.

In Figur 2 ist beispielhaft eine Einteilung in einen ersten Abschnitt 20a und einen zweiten Abschnitt 20b angedeutet, wobei in diesem Beispiel der erste Abschnitt stromab bezogen auf den zweiten Abschnitt angeordnet ist.

Wie oben bereits erläutert, sind erster und zweiter Abschnitt jeweils abhängig davon, wo der Druck überwacht wird. Wenn also der Drucksensor 6 beispielsweise im Bereich der Leitung 1 angeordnet wäre, so wäre der erste Abschnitt stromauf bezogen auf den zweiten Abschnitt angeordnet. Mit anderen Worten kann der erste Abschnitt (und entsprechend der zweite Abschnitt) eingangsseitig oder ausgangsseitig angeordnet sein.

Weitere Vorteile und Merkmale gemäß der vorliegenden Offenbarung werden anhand der folgenden Beschreibung ersichtlich.

Die vorliegende Offenbarung beschreibt ein Verfahren zur Überprüfung eines Wärmetauschers eines Dialysegerätes auf Dichtigkeit, wobei Dialysierflüssigkeitsseitig ein Druck appliziert wird. Gängige Dialysemaschinen beinhalten einen Wärmetauscher, der die Wärme des abfließenden Dialysats nutzt, um das zufließende und im Vergleich zum Dialysat kältere Permeat vorzuwärmen. Von diesem Wärmetauscher wird gefordert, dass er inhärent sicher sein soll. Es wird demnach davon ausgegangen, dass es zu keinen Undichtigkeiten kommen kann. Im Falle eines Defekts bzw. einer Undichtigkeit im Wärmetauscher kann ein Übergang von verbrauchtem Dialysat auf die frische Permeat- bzw. Dialysierflüssigkeitsseite von gängigen Dialysemaschinen nicht erkannt werden. Dies würde zu einer Kontamination der Dialysierflüssigkeitsseite führen, die es zu vermeiden gilt.

Mögliche Verfahren und Vorrichtungen gemäß der vorliegenden Offenbarung wurden oben bereits erläutert, insbesondere bezugnehmend auf Figuren 1 und 2.

Im Folgenden werden, wieder auf die Figur 2 Bezug nehmend, weitere nichtbeschränkende Beispiele im Detail erläutert.

Figur 2 zeigt schematisch und vereinfacht ein Beispiel für eine Blutbehandlungsvorrichtung 100, hier am Beispiel eines Dialysesystems. Im Folgenden wird kurz ein Beispiel für den Betrieb eines Dialysesystems, beispielsweise des in Figur 2 gezeigten Dialysesystems beschrieben:
Durch das Ventil 2 gelangt hochreines Wasser, sogenanntes Permeat, in das Dialysesystem und durchläuft dort den Wärmetauscher 13. Im Wärmetauscher 13 findet eine Wärmeübertrag statt. Das Permeat nimmt hierbei Wärmeenergie des Dialysats auf, welches durch die Leitung 12 und anschließend durch den Wärmetauscher 13 aus dem Flüssigkeitskreislauf hinausströmt, und wird auf diese Weise vorgewärmt.

Der Wärmetauscher 13 kann sich innerhalb oder außerhalb eines Dialysegeräts befinden (beispielsweise bezogen auf ein Gehäuse).

Durch die Leitung 1 fließt das Permeat weiter.

Optional kann das Permeat durch eine Temperiervorrichtung (nicht dargestellt) fließen, wo das Wasser auf die benötigte Temperatur erwärmt wird. Der Wärmetauscher 13 kann optional als integraler Bestandteil der Temperiervorrichtung ausgebildet sein.

Gefördert durch eine erste Fördervorrichtung 3 strömt das Wasser bzw. das Gemisch aus Wasser und mindestens einem Konzentrat durch die Bilanzvorrichtung 10 und das Dialysatoreingangsventil 4 in die Spülbrücke 5. Diese verbindet bei einer Dialysevorrichtung, welche noch nicht mit einem Dialysator gerüstet ist, wasserseitigen Eingang mit Ausgang anstelle eines Dialysators. Statt der Spülbrücke 5 kann also auch ein Dialysator vorgesehen sein. Alternativ kann durch geeignete Ventilstellung das Wasser/Gemisch durch den Bypass geleitet werden.

Die zweite Fördervorrichtung 9 fördert das Dialysat in die Bilanzvorrichtung 10, welche einen kontrollierten Entzug von überschüssigem Wasser mit Hilfe der Fördervorrichtung 11 erlaubt. Von dort gelangt es durch die Leitung 12 zum Wärmetauscher 13 und anschließend in den Abfluss.

Die Bilanzvorrichtung ist optional.

Weitere gängige Bestandteile von Blutbehandlungsvorrichtungen, insbesondere Dialysesystemen, wie z.B. Blutleckdetektor, Temperatursensoren etc., sind zugunsten der Übersichtlichkeit in Figur 2 nicht dargestellt.

Im Falle eines defekten Wärmetauschers 13 kann es passieren, dass Dialysat aus der Leitung 12 in die permeat- bzw. dialysierflüssigkeitsführende Leitung 1 strömt und diese kontaminiert. Da dies außerdem unerkannt zu einer Minderung der Dialyseeffizienz führen würde, beschreibt die vorliegende Erfindung Verfahren zur Detektion eines Defekts bzw. einer Undichtigkeit des Wärmetauschers 13.

In einem ersten Beispiel wird mit Hilfe der Fördervorrichtungen 3 und 9 Flüssigkeit in den Kreislauf des Dialysesystems 100 gefördert. Die Bilanzierungsvorrichtung befindet sich dabei im Durchflussmodus während Fördervorrichtung 11 ebenfalls eine definierte Position einnimmt. Die Temperatur im Dialysesystem 100 ist Jederzeit bekannt bzw. wird mit Hilfe der Temperiervorrichtung auf einen definierten Wert eingestellt. Ventil 14 befindet sich in dieser Ausführungsform im Inneren des Dialysegerätes und kann zum Zweck der Druckerzeugung geschlossen werden. Wird bei geschlossenen Ventil 14 weiter Flüssigkeit in das Gerät gefördert, so kann ein Druckanstieg an Messeinrichtung 6 detektiert werden. Hat der Druck an Messeinrichtung 6 einen definierten Wert erreicht, werden die Ventile 8 und 4 geschlossen und die Fördervorrichtungen 9 und 3 gestoppt.

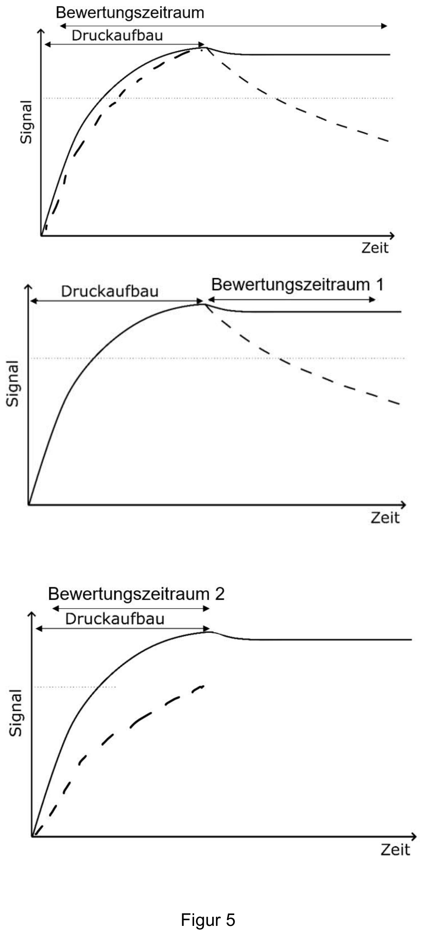

Hat sich der gemessene Druckwert stabilisiert und das System eingeschwungen, beginnt ein Bewertungszeitraum, in dem die Stabilität des aufgebauten Druckes überwacht wird. Sinkt der Druck innerhalb dieses definierten Zeitraumes unter einen definierten Wert (vgl. gepunktete Linie in Figur 5), so wird der Wärmetauscher 13 als defekt klassifiziert (gestrichelte Linie in Figur 5). Das Maximum der Ableitung des gemessenen Drucksignals kann hierbei als Indikator für die Größe der Leckage dienen und zur Größe des Lecks in Beziehung gesetzt werden.

Dies kann ggf. mithilfe einer im Dialysegerät hinterlegten Logik oder Lookup-Table geschehen. Dies kann ggf. auch basierend auf einer Berechnung geschehen, solange der Druck permanent gemessen wird und die Temperatur bekannt ist.

Während das obige Beispiel in Bezug auf den Druckwert beschrieben wird, kann das Verfahren alternativ mit dem Betrag des Druckwerts durchgeführt werden. Beispielsweise kann das Drucksignal mit dem Druckwert korrelieren oder das Drucksignal kann mit dem Betrag des Druckwerts korrelieren. Beispielsweise könnten dann beim Druckaufbau und beim Unterdruckaufbau ähnliche Signalverläufe beobachtet werden.

Der Drucksensor 6 befindet sich in dem in Figur 2 gezeigten Beispiel zwischen Spülbrücke 5 und Dialysatorausgangsventil 7. Der Drucksensor kann sich aber auch an anderen Stellen, beispielsweise stromab der Bilanziereinrichtung 10 und der Fördereinrichtung 11 in Leitung 12 näher am Wärmetauscher 13 befinden.

In einem weiteren Beispiel kann bei geschlossenen Ventilen 2, 7 und 8 mit einer der beiden Konzentratpumpen 3a, 3b ein Druck aufgebaut werden. Kann in einer vordefinierten Zeit kein Druck aufgebaut werden oder fällt dieser nach Pumpenstopp zu schnell ab, deutet dies ebenfalls auf eine Leckage im Wärmetauscher 13 hin.

In einem weiteren Beispiel kann bei geschlossenen Ventilen 2, 14, 4 und 8 mit einer der beiden Konzentratpumpen 3a, 3b ein Druck aufgebaut werden. Wird bei offenem Ventil 7 ein Druckanstieg an Drucksensor 6 registriert, deutet dies ebenfalls auf eine Leckage im Wärmetauscher 13 hin.

Figur 3 zeigt ein System gemäß der vorliegenden Offenbarung. Die dargestellte Anordnung vermeidet einen internen Verbau des Ventils 14 innerhalb des Dialysegeräts 101, was aus Produktkostengründen wünschenswert sein kann. Das grundlegende Verfahren entspricht Figur 2 jedoch mit dem Unterschied, dass Ventil 14 portabel ausgeführt ist und beispielsweise von einem Servicetechniker bei einer STK eingeschlaucht werden kann oder als Quetschventil ausgeführt ist, so dass es nur temporär Verwendung findet. Dazu ist Ventil 14 mit einer Energieversorgungseinrichtung 17 ausgestattet und mit einer Synchronisationsvorrichtung 16, so dass während eines Tests der korrekte Zeitpunkt zum Schließen durch das Dialysegerät an Ventil 14 kommuniziert werden kann. Dies kann per drahtloser oder kabelgebundener Kommunikation 18 erfolgen sofern das Dialysegerät 101 mit einer entsprechenden Kommunikationsvorrichtung (nicht dargestellt) versehen ist.

Figur 4 zeigt eine Anordnung, die einen internen Verbau des Ventils 14 innerhalb des Dialysegeräts 101 vermeidet, was aus Produktkostengründen wünschenswert sein kann. Das Ventil wird jedoch nicht direkt vom Dialysegerät 101 gesteuert, sondern der Ablauf im Dialysegerät und die korrekten Schaltzeitpunkte für Ventil 14 werden mit Hilfe einer Kontrollvorrichtung 19 synchronisiert. Bei der Kontrollvorrichtung kann es sich beispielsweise um den PC eines Servicetechnikers handeln, der durch die Verwendung einer Wartungssoftware sowohl Dialysegerät steuert als auch Ventil 14 etc. Alternativ kann die Kontrollvorrichtung 19 beispielsweise genau wie Ventil 14 Teil einer Produktionsinfrastruktur sein.

Obwohl die Erfindung in den Zeichnungen und der vorstehenden Beschreibung detailliert dargestellt und beschrieben ist, sind diese Darstellungen und Beschreibungen als beispielhaft und nicht einschränkend zu betrachten. Die Erfindung ist nicht auf die offengelegten Ausführungsformen beschränkt. In Anbetracht der vorstehenden Beschreibung und der Zeichnungen wird es für den Fachmann offensichtlich sein, dass im Rahmen der Erfindung, wie sie in den Ansprüchen definiert ist, verschiedene Modifikationen vorgenommen werden können.

Bezugszeichen in den oben beschriebenen Beispielen:
1 Leitung eingangsseitig
2 Ventil Wassereingang
3 Flusspumpe Eingang
3a, 3b Konzentratpumpen
4 Ventil Dialysatoreingang
5 Spülbrücke
6 Drucksensor
7 Ventil Dialysatorausgang
8 Ventil Bypass
9 Flusspumpte Ausgang
10 Bilanzierungsvorrichtung
11 Fördervorrichtung
12 Leitung wasserausgangsseitig
13 Wärmetauscher
14 Ventil Abfluss
15 Leitung Abfluss (außerhalb)
16 Synchronisationsvorrichtung
17 Energieversorgungseinrichtung
18 Kommunikation
19 Kontrollvorrichtung
20 Flüssigkeitskreislauf
20a erster Abschnitt
20b zweiter Abschnitt
21 Rechensystem
100 Blutbehandlungsvorrichtung
101 Dialysegerät
200 System

## Patentansprüche

1. Verfahren zur Dichtigkeitsprüfung eines Wärmetauschers (13) einer Blutbehandlungsvorrichtung (100), umfassend
Überwachen (S11) des zeitlichen Verlaufs eines mittels eines Drucksensors (6) erfassten Druckes in einem ersten Abschnitt (20a) eines Flüssigkeitskreislaufs (20) der Blutbehandlungsvorrichtung (100) in einem Bewertungszeitraum;
Überprüfen (S12) mittels eines Rechensystems (21), ob der zeitliche Verlauf des Druckes in dem Bewertungszeitraum ein oder mehrere vorbestimmte Kriterien erfüllt; und
Feststellen (S13) einer Undichtigkeit des Wärmetauschers (13) für den Fall, dass der zeitliche Verlauf das bzw. die vorbestimmten Kriterien erfüllt,
wobei der Wärmetauscher (13) zwei Strömungswege umfassend einen ersten Strömungsweg, der mit dem ersten Abschnitt (20a) des Flüssigkeitskreislaufs (20) fluidisch verbunden ist, und einen zweiten Strömungsweg, der mit einem zweiten Abschnitt (20b) des Flüssigkeitskreislaufs (20) fluidisch verbunden ist, aufweist und der Flüssigkeitskreislauf (20) über die Strömungswege mit einem Zulauf und einen Ablauf verbunden ist,
wobei der erste Abschnitt (20a) des Flüssigkeitskreislaufs (20) während des Bewertungszeitraums unter Verwendung von einem oder mehreren Schaltelementen von dem zweiten Abschnitt (20b) des Flüssigkeitskreislaufs (20) und/oder dem Zulauf und/oder dem Ablauf fluidisch getrennt ist, und
wobei das Überwachen des zeitlichen Verlaufs in dem Bewertungszeitraum das Überwachen des zeitlichen Verlaufs in einem ersten Bewertungszeitraum während eines Fördervorrichtungsbetriebs zum Druckaufbau/Unterdruckaufbau in dem ersten Abschnitt (20a) und/oder dem zweiten Abschnitt (20b) des Flüssigkeitskreislaufs (20) und/oder wobei das Überwachen des zeitlichen Verlaufs in einem zweiten Bewertungszeitraum nach einem Druckaufbau/Unterdruckaufbau in dem ersten Abschnitt (20a) des Flüssigkeitskreislaufs (20) umfasst.

2. Verfahren nach Anspruch 1,
wobei der erste Abschnitt (20a) des Flüssigkeitskreislaufs (20) in dem Bewertungszeitraum unter Verwendung von einem oder mehreren Schaltelementen von dem zweiten Abschnitt (20b) des Flüssigkeitskreislaufs (20) und dem Ablauf fluidisch getrennt ist und insbesondere der zweite Abschnitt (20b) fluidisch mit dem Zulauf verbunden ist,
wobei das Überwachen in dem zweiten Bewertungszeitraum durchgeführt wird und das vorbestimmte Kriterium umfasst, dass ein Druckanstieg/Druckabfall des erfassten Druckes im zweiten Bewertungszeitraum von einem Referenzdruckanstieg/Referenzdruckabfall abweicht, wobei der Druckanstieg/Druckabfall insbesondere größer ist als der Referenzdruckanstieg/Referenzdruckabfall, und/oder
wobei das Überwachen in dem ersten Bewertungszeitraum während eines Fördervorrichtungsbetriebs einer im ersten Abschnitt (20a) angeordneten ersten Fördervorrichtung (9) und/oder einer im zweiten Abschnitt (20b) angeordneten zweiten Fördervorrichtung (3, 3a, 3b) zum Druckaufbau/Unterdruckaufbau in dem ersten Abschnitt (20a) durchgeführt wird und das vorbestimmte Kriterium umfasst, dass ein Druckanstieg/Druckabfall des erfassten Druckes im ersten Bewertungszeitraum von einem Referenzdruckanstieg/Referenzdruckabfall abweicht, wobei der Druckanstieg/Druckabfall insbesondere kleiner ist als der Referenzdruckanstieg/Referenzdruckabfall.

3. Verfahren nach Anspruch 1,
wobei der erste Abschnitt (20a) des Flüssigkeitskreislaufs (20) in dem Bewertungszeitraum unter Verwendung von einem oder mehreren Schaltelementen von dem zweiten Abschnitt (20b) des Flüssigkeitskreislaufs (20) und dem Ablauf fluidisch getrennt ist,
wobei das Überwachen in dem ersten Bewertungszeitraum während eines Fördervorrichtungsbetriebs einer im zweiten Abschnitt (20b) angeordneten zweiten Fördervorrichtung (3, 3a, 3b) zum Druckaufbau/Unterdruckaufbau in dem zweiten Abschnitt (20b) durchgeführt wird und das vorbestimmte Kriterium umfasst, dass ein Druckanstieg/Druckabfall des erfassten Druckes im ersten Bewertungszeitraum von einem Referenzdruckanstieg/Referenzdruckabfall abweicht, wobei der Druckanstieg/Druckabfall insbesondere größer ist als der Referenzdruckanstieg/Referenzdruckabfall.

4. Verfahren nach Anspruch 1,
wobei der erste Abschnitt (20a) des Flüssigkeitskreislaufs in dem Bewertungszeitraum unter Verwendung von einem oder mehreren Schaltelementen von dem zweiten Abschnitt (20b) des Flüssigkeitskreislaufs und dem Zulauf fluidisch getrennt ist und insbesondere der zweite Abschnitt (20b) fluidisch mit dem Ablauf verbunden ist,
wobei das Überwachen in dem zweiten Bewertungszeitraum durchgeführt wird und das vorbestimmte Kriterium umfasst, dass ein Druckanstieg/Druckabfall des erfassten Druckes im zweiten Bewertungszeitraum von einem Referenzdruckanstieg/Referenzdruckabfall abweicht, wobei der Druckanstieg/Druckabfall insbesondere größer ist als der Referenzdruckanstieg/Referenzdruckabfall, und/oder
wobei das Überwachen in dem ersten Bewertungszeitraum während eines Fördervorrichtungsbetriebs einer im ersten Abschnitt (20a) angeordneten zweiten Fördervorrichtung (3, 3a, 3b) zum Druckaufbau/Unterdruckaufbau in dem ersten Abschnitt (20a) durchgeführt wird und das vorbestimmte Kriterium umfasst, dass ein Druckanstieg/Druckabfall des erfassten Druckes im ersten Bewertungszeitraum von einem Referenzdruckanstieg/Referenzdruckabfall abweicht, wobei der Druckanstieg/Druckabfall insbesondere kleiner ist als der Referenzdruckanstieg/Referenzdruckabfall.

5. Verfahren nach Anspruch 2 oder 3, wobei der erste Abschnitt (20a) bezogen auf den zweiten Abschnitt (20b) stromabwärts angeordnet ist.

6. Verfahren nach Anspruch 4, wobei der erste Abschnitt (20a) bezogen auf den zweiten Abschnitt (20b) stromaufwärts angeordnet ist und/oder wobei die zweite Fördervorrichtung (3, 3a, 3b) bezogen auf den Drucksensor (6) stromaufwärts angeordnet ist.

7. Verfahren nach Anspruch 2 oder 3, wobei die Fördervorrichtung bezogen auf den Drucksensor (6) stromaufwärts oder stromabwärts angeordnet ist und/oder wobei die erste Fördervorrichtung (9) bezogen auf den Drucksensor (6) stromabwärts angeordnet ist.

8. Verfahren nach einem der vorangegangen Ansprüche, wobei im zweiten Bewertungszeitraum der Fördervorrichtungsbetrieb, insbesondere der ersten Fördervorrichtung (9) und/oder der zweiten Fördervorrichtung (3, 3a, 3b), eingestellt ist.

9. Verfahren nach einem der vorangegangenen Ansprüche, wobei die zweite Fördervorrichtung (3, 3a, 3b) eine oder mehrere Konzentratpumpen (3a, 3b) umfasst, die zum Zuführen von Konzentrat in eine Flüssigkeit im Flüssigkeitskreislauf (20) ausgebildet sind.

10. Verfahren nach einem der vorangegangenen Ansprüche, wobei der erste Abschnitt (20a) bzw. der zweite Abschnitt (20b) mittels eines externen Absperrelements (14) fluidisch vom Ablauf trennbar sind, insbesondere wobei das externe Absperrelement (14) mittels einer Steuervorrichtung der Blutbehandlungsvorrichtung (100) und/oder mittels einer externen Steuervorrichtung (19) steuerbar ist, wobei die externe Steuervorrichtung zum Steuern des externen Absperrelements (14) und optional der Blutbehandlungsvorrichtung (100) ausgebildet ist.

11. Verfahren nach Anspruch 10, wobei ein Zeitpunkt zum Öffnen und/oder Schließen des externen Absperrelements (14) mittels einer Synchronisationsvorrichtung (16) von der Blutbehandlungsvorrichtung (100) an das externe Absperrelement (14) kommuniziert wird.

12. Blutbehandlungsvorrichtung (100) umfassend
einen Flüssigkeitskreislauf (20) und einen Wärmetauscher (13), wobei der Wärmetauscher (13) zwei Strömungswege umfassend einen ersten Strömungsweg, der mit einem ersten Abschnitt (20a) des Flüssigkeitskreislaufs (20) fluidisch verbunden ist, und einen zweiten Strömungsweg, der mit einem zweiten Abschnitt (20b) des Flüssigkeitskreislaufs (20) fluidisch verbunden ist, aufweist und der Flüssigkeitskreislauf (20) über die Strömungswege mit einem Zulauf und einen Ablauf verbunden ist;
einen Drucksensor (6), der zum Erfassen eines Druckes in dem ersten Abschnitt (20a) des Flüssigkeitskreislaufs (20) der Blutbehandlungsvorrichtung (100) ausgebildet ist;
ein Rechensystem (21), das zum Überwachen eines zeitlichen Verlaufs des mittels des Drucksensors (6) erfassten Druckes in einem Bewertungszeitraum, in dem die Blutbehandlungsvorrichtung (100) eine Prüfkonfiguration aufweist, zum Überprüfen, ob der zeitliche Verlauf des Druckes in dem Bewertungszeitraum ein oder mehrere vorbestimmte Kriterien erfüllt, und zum Feststellen einer Undichtigkeit des Wärmetauschers (13) für den Fall, dass der zeitliche Verlauf das bzw. die vorbestimmten Kriterien erfüllt, ausgebildet ist,
wobei der erste Abschnitt (20a) des Flüssigkeitskreislaufs (20) in der Prüfkonfiguration unter Verwendung von einem oder mehreren Schaltelementen von dem zweiten Abschnitt (20b) des Flüssigkeitskreislaufs (20) und/oder dem Zulauf und/oder dem Ablauf fluidisch getrennt ist, und
wobei das Überwachen des zeitlichen Verlaufs in dem Bewertungszeitraum das Überwachen des zeitlichen Verlaufs in einem ersten Bewertungszeitraum während eines Fördervorrichtungsbetriebs zum Druckaufbau/Unterdruckaufbau in dem ersten Abschnitt (20a) und/oder dem zweiten Abschnitt (20b) des Flüssigkeitskreislaufs (20) und/oder das Überwachen des zeitlichen Verlaufs in einem zweiten Bewertungszeitraum nach einem Druckaufbau/Unterdruckaufbau in dem ersten Abschnitt (20a) des Flüssigkeitskreislaufs (20) umfasst.

13. Blutbehandlungsvorrichtung (100) nach Anspruch 12,
wobei in einer ersten Prüfkonfiguration der Blutbehandlungsvorrichtung (100) der erste Abschnitt (20a) des Flüssigkeitskreislaufs (20) unter Verwendung von einem oder mehreren Schaltelementen von dem zweiten Abschnitt (20b) des Flüssigkeitskreislaufs (20) und dem Ablauf fluidisch getrennt ist und insbesondere der zweite Abschnitt (20b) fluidisch mit dem Zulauf verbunden ist,
wobei die Blutbehandlungsvorrichtung (100) ausgebildet ist, das Überwachen in dem zweiten Bewertungszeitraum durchzuführen, und das vorbestimmte Kriterium umfasst, dass ein Druckanstieg/Druckabfall des erfassten Druckes im zweiten Bewertungszeitraum von einem Referenzdruckanstieg/Referenzdruckabfall abweicht, wobei der Druckanstieg/Druckabfall insbesondere größer ist als der Referenzdruckanstieg/Referenzdruckabfall, und/oder
wobei die Blutbehandlungsvorrichtung (100) ausgebildet ist, das Überwachen in dem ersten Bewertungszeitraum während eines Fördervorrichtungsbetriebs einer im ersten Abschnitt (20a) angeordneten ersten Fördervorrichtung (9) und/oder einer im zweiten Abschnitt (20b) angeordneten zweiten Fördervorrichtung (3, 3a, 3b) zum Druckaufbau/Unterdruckaufbau in dem ersten Abschnitt (20a) durchzuführen, und das vorbestimmte Kriterium umfasst, dass ein Druckanstieg/Druckabfall des erfassten Druckes im ersten Bewertungszeitraum von einem Referenzdruckanstieg/Referenzdruckabfall abweicht, wobei der Druckanstieg/Druckabfall insbesondere kleiner ist als der Referenzdruckanstieg/Referenzdruckabfall,
wobei in der ersten Prüfkonfiguration insbesondere der erste Abschnitt (20a) bezogen auf den zweiten Abschnitt (20b) stromabwärts angeordnet ist und/oder die Fördervorrichtung bezogen auf den Drucksensor (6) stromaufwärts oder stromabwärts angeordnet ist und/oder wobei die erste Fördervorrichtung (9) bezogen auf den Drucksensor (6) stromabwärts angeordnet ist.

14. Blutbehandlungsvorrichtung (100) nach Anspruch 12,
wobei in einer zweiten Prüfkonfiguration der Blutbehandlungsvorrichtung (100) der erste Abschnitt (20a) des Flüssigkeitskreislaufs (20) unter Verwendung von einem oder mehreren Schaltelementen von dem zweiten Abschnitt (20b) des Flüssigkeitskreislaufs (20) und dem Ablauf fluidisch getrennt ist,
wobei die Blutbehandlungsvorrichtung (100) ausgebildet ist, das Überwachen in dem ersten Bewertungszeitraum während eines Fördervorrichtungsbetriebs einer im zweiten Abschnitt (20b) angeordneten zweiten Fördervorrichtung (3, 3a, 3b) zum Druckaufbau/Unterdruckaufbau in dem zweiten Abschnitt (20b) durchzuführen, und das vorbestimmte Kriterium umfasst, dass ein Druckanstieg/Druckabfall des erfassten Druckes im ersten Bewertungszeitraum von einem Referenzdruckanstieg/Referenzdruckabfall abweicht, wobei der Druckanstieg/Druckabfall insbesondere größer ist als der Referenzdruckanstieg/Referenzdruckabfall,
wobei in der zweiten Prüfkonfiguration insbesondere der erste Abschnitt (20a) bezogen auf den zweiten Abschnitt (20b) stromabwärts angeordnet ist.

15. Blutbehandlungsvorrichtung (100) nach Anspruch 12,
wobei in einer dritten Prüfkonfiguration der Blutbehandlungsvorrichtung (100) der erste Abschnitt (20a) des Flüssigkeitskreislaufs (20) unter Verwendung von einem oder mehreren Schaltelementen von dem zweiten Abschnitt (20b) des Flüssigkeitskreislaufs (20) und dem Zulauf fluidisch getrennt ist und insbesondere der zweite Abschnitt (20b) fluidisch mit dem Ablauf verbunden ist,
wobei die Blutbehandlungsvorrichtung (100) ausgebildet ist, das Überwachen in dem zweiten Bewertungszeitraum durchzuführen, und das vorbestimmte Kriterium umfasst, dass ein Druckanstieg/Druckabfall des erfassten Druckes im zweiten Bewertungszeitraum von einem Referenzdruckanstieg/Referenzdruckabfall abweicht, wobei der Druckanstieg/Druckabfall insbesondere größer ist als der Referenzdruckanstieg/Referenzdruckabfall, und/oder
wobei die Blutbehandlungsvorrichtung (100) ausgebildet ist, das Überwachen in dem ersten Bewertungszeitraum während eines Fördervorrichtungsbetriebs einer im ersten Abschnitt (20a) angeordneten zweiten Fördervorrichtung (3, 3a, 3b) zum Druckaufbau/Unterdruckaufbau in dem ersten Abschnitt (20a) durchzuführen, und das vorbestimmte Kriterium umfasst, dass ein Druckanstieg/Druckabfall des erfassten Druckes im ersten Bewertungszeitraum von einem Referenzdruckanstieg/Referenzdruckabfall abweicht, wobei der Druckanstieg/Druckabfall insbesondere kleiner ist als der Referenzdruckanstieg/Referenzdruckabfall,
insbesondere wobei in der dritten Prüfkonfiguration der erste Abschnitt (20a) bezogen auf den zweiten Abschnitt (20b) stromaufwärts angeordnet ist und/oder wobei die zweite Fördervorrichtung (3, 3a, 3b) bezogen auf den Drucksensor (6) stromaufwärts angeordnet ist und/oder wobei die Fördervorrichtung bezogen auf den Drucksensor (6) stromaufwärts oder stromabwärts angeordnet ist und/oder wobei die erste Fördervorrichtung (9) bezogen auf den Drucksensor (6) stromabwärts angeordnet ist.

16. System (200) umfassend die Blutbehandlungsvorrichtung (100) nach einem der Ansprüche 12 bis 15,
wobei das System (200) ein externes Absperrelement (14) und eine Steuervorrichtung der Blutbehandlungsvorrichtung (100) und/oder eine externe Steuervorrichtung (19) umfasst und wobei der erste Abschnitt (20a) bzw. der zweite Abschnitt (20b) mittels des externen Absperrelements (14) fluidisch vom Ablauf trennbar sind, insbesondere wobei das externe Absperrelement (14) mittels der Steuervorrichtung der Blutbehandlungsvorrichtung (100) und/oder mittels der externen Steuervorrichtung (19) steuerbar ist, wobei die externe Steuervorrichtung zum Steuern des externen Absperrelements (14) und optional der Blutbehandlungsvorrichtung (100) ausgebildet ist, und/oder
wobei das System (200) ein/das externe Absperrelement (14) und eine Synchronisationsvorrichtung (16) umfasst, wobei ein Zeitpunkt zum Öffnen und/oder Schließen des externen Absperrelements (14) mittels der Synchronisationsvorrichtung (16) von der Blutbehandlungsvorrichtung (100) an das externe Absperrelement (14) kommuniziert wird.
